# EUROPEAN PATENT APPLICATION

(11) **EP 3 741 866 A1**
(43) Date of publication of application: **25.11.2020**
(21) Application number: 20175541.0
(22) Date of filing: 19.05.2020
(51) Int. Cl.: C12Q 1/08, B01L 3/00, C12Q 1/18

(54) **KIT AND METHOD FOR ANTIBIOTICS SUSCEPTIBILITY TESTING WITH THE AGAR DILUTION METHOD**

(30) Priority: 20.05.2019 IT 201900007007
(71) Applicant: Liofilchem S.r.l., 64026 Roseto Degli Abruzzi (TE) (IT)
(72) Inventor: BROCCO, Silvio, 64026 Roseto degli Abruzzi (TE) (IT)
(74) Representative: Primiceri, Maria Vittoria

(57) **Abstract**

The invention relates to a kit and method for antibiotics susceptibility testing. The test and method are performed by using the agar dilution procedure.

More particularly the invention relates to a kit and method for assessing the minimum inhibitory concentration (M.I.C.) of antibiotics belonging to various classes, such as for example penicillins, cephalosporins, carbapenems, aminoglycosides, quinolones, fosfomycin, lincosamides, macrolides, glycopeptides, tetracyclines, azoles and echinocnidines on plates comprising at least one detection assay vessel in form of an agarized well. Still more particularly, this invention relates to a kit for facilitating the carrying out of such method.

## Description

### Field of invention

The present invention relates to a kit and method for antibiotics susceptibility testing. The test and method are performed by using the agar dilution procedure. More particularly the invention relates to a method for assessing the minimum inhibitory concentration (M.I.C.) of an antibiotic on plates comprising at least one detection assay vessel in form of an agarized well, as defined below. Still more particularly, this invention relates to a kit for facilitating the carrying out of such method.

### Background art

With an increase in the numbers of bacterial isolates resistant to first-line antibiotics, there has been a revival in the use of older drugs. Although several commercial tests based on different techniques have been developed, Agar Dilution (AD) is so far considered the best method for performing susceptibility test to some antibiotics, including fosfomycin.

Agar dilution method is the gold standard to measure quantitatively the *in vitro* activity of some antibiotics including fosfomycin against a given bacterial isolate. To perform the tests, a series of plates is prepared with an agar medium to which various concentrations of the antimicrobial agent are added. The plates are then inoculated with a standardized suspension of the test organism. After incubation at 35 ± 2°C, the tests are examined and the MIC is determined. However the final result is significantly influenced by methodology, which must be carefully controlled if reproducible results (intra-laboratory and inter-laboratory) are to be achieved.

The reference standard for agar dilution methods are described in Clinical and Laboratory Standards Institute, January 2009, M07-A8, Vol. 29 n.2 and the only approved MIC method for testing antibiotics, among which fosfomycin, is agar dilution using agar media supplemented with 25µg/mL glucose-6-phosphate (Laboratory Standards Institute, M100, 29th ed. January 2019). However, due to their complexity, gold standard susceptibility methodologies such as agar dilution susceptibility testing are rarely, if ever, performed by hospital-based clinical microbiology laboratories. These standard susceptibility methodologies require a large number of pipetting steps to create an antimicrobial doubling dilution series that can be tested for antimicrobial effects and minimal inhibitory concentration (MIC) determination. The time and manual labor required for this type of testing, even for a few isolates, is prohibitive due to the fast-paced workflow required in clinical laboratories.

Not to mention that the execution of this high number of operations entails an equally high risk of contamination which is greater the greater the number of agarized petri dishes to be prepared. In general, laboratories use 90mm diameter petri dishes to have adequate support for the determination of the MIC and the number of dilutions to be made is at least six dilutions and can even reach fifteen. Correspondingly, the number of agarized plates to be prepared entails a high risk of contamination, due to the bacterial growth naturally present in the air, so that the devices made in-house after 24 hours are no longer usable if polluted, and require production and corresponding extemporaneous use. Not to mention that the plates so prepared must be carefully indexed, identified in such a way that the corresponding concentration of antibiotic is identified.

The operations that the analysis laboratories currently have to carry out are manifold and are connected with the preparation of the mother solution containing the antibiotic according to the gold standard, carrying out the necessary dilutions, mixing with agar and preparing it in the individual plates. Each of these operations is a potential source not only of contamination, because each capsule needs careful handling, but is also a source of errors, because each plate must be prepared in its own way and individually labeled and, as you know, as many as operations are greater to achieve a result, the greater the margin of error that this result entails.

Therefore, hospital-based clinical laboratories tend to employ easier alternatives to gold standard susceptibility methodologies.

At present the only reliable option available to most clinical microbiology laboratories is to send isolates to a "reference laboratory" for dilution MIC testing. Furthermore, antibiotics susceptibility testing is complicated and prone to error and many scientific publications indicate that different methods from agar dilution for testing antibiotics such as fosfomycin are not reliable.

Accordingly, there remains a critical need to test antibiotics in general and fosfomycin in particular in pre-made panels in order to allow to avoid or minimize contamination and human errors.

### Summary of invention

It has now been found that the miniaturization in wells for testing susceptibility to antibiotics in general and to fosfomycin in particular is possible and does not negatively affect the results. It is therefore an object of this invention to provide a simple and stable device for assessing the antibacterial activity of the antibiotic. It is another object of this invention to provide a simple device for assessing the activity of the antibiotic according to the agar dilution procedure on determined amounts of agar compositions placed in wells, called in the following agarized wells.

The method of the invention comprises preparing a device comprising a plurality of agarized wells with serial two-fold dilutions of antibiotic concentration, so that the user inoculates the test bacteria into the agarized wells, determining MIC activity on the basis of the presence or absence of the formation of macroscopic bacterial colonies after incubation of the inoculated wells for a fixed period of time and under predetermined laboratory conditions. These predetermined conditions entail the automated conduct of the operations until a single plate is adopted in which a plurality of agarized wells with serial dilutions are doubled to double the concentration of antibiotic. These operations, being carried out in a controlled atmosphere until the plate is packaged and sealed, so that it leaves the production line ready for use, and allow to obtain a standardized device for performing tests in a repeatable and reproducible way both intra-laboratory and inter-laboratory.

Still another object of the invention is to provide a detection assay vessel in form of an agarized well, as defined below for carrying out of such method.

These and other objects and advantages of the invention will become apparent from the following detailed description of the invention.

### Brief description of figures

The objects of the invention are further illustrated by the accompanying drawings, in which:
Fig. 1 schematically shows the antibiotic Agar Dilution Workflow;

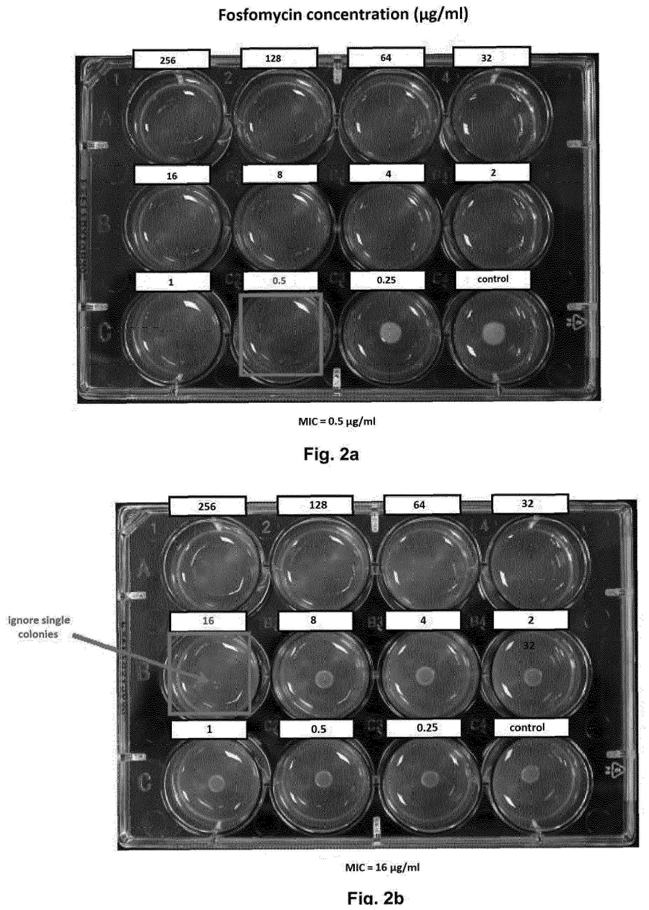

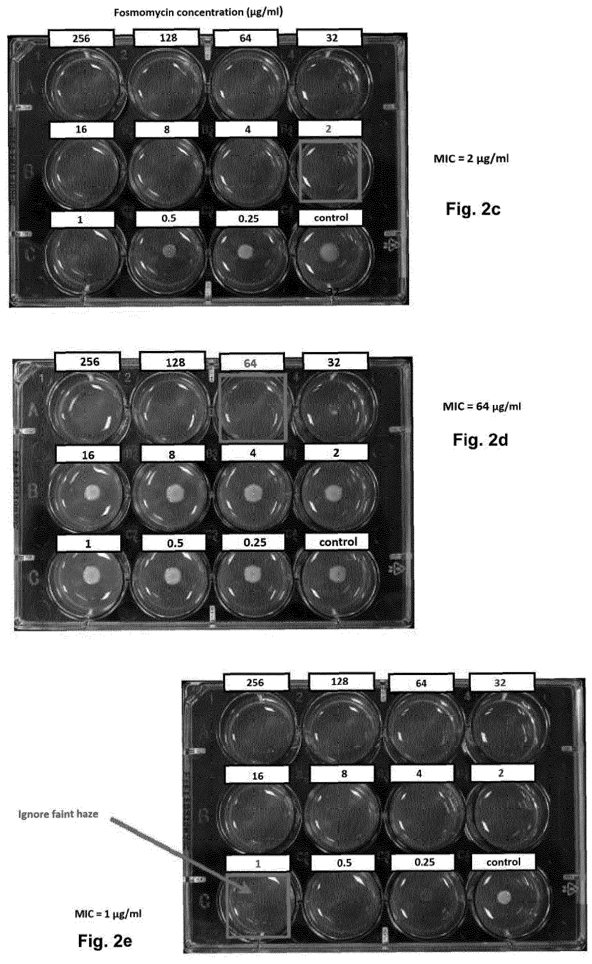

Fig. 2 schematically shows different antibiotic Agar Dilution tests in which different MIC values have been observed. In Fig. 2a a MIC of 0.5µg/ml; in Fig. 2b a MIC of 16µg/ml; in Fig. 2c a MIC of 2µg/ml; in Fig. 2d a faint haze is observed at 1µg/ml.

The figures are given in order to more completely illustrate the invention but should not be considered as limitative of the scope thereof.

### Detailed description of invention

According to the inventions by the terms "agarized well/s" we mean one or a series of wells filled with a given amount of agar compositions prepared according to (Ref 1) and containing scalar quantities of antibiotic.

Preferably each well has U or V or flat bottom, depth 15-17mm, diameter 20-25mm, working volume 2-4ml, growth area 2-4cm².

As already noted, the invention relates to a method for assessing the antibacterial activity of antibiotic.

The process for preparing each plate comprising a plurality of agarized wells with doubled serial dilutions of antibiotic according to the international guidelines that constitute the gold standard for carrying out the method for evaluating the antibacterial activity of the antibiotic (Clinical and Laboratory Standards Institute, January 2009, M07-A8, vol. 29 n.2) comprises the stages of:
- prepare the antibiotic solution and its dilutions;
- prepare the agarized soil (Mueller Hinton), sterilize it and aliquot it in 12 bottles;
- add the respective antibiotic dilution to each bottle;
- distribute the antibiotic solutions in the respective wells of the device.

These stages are conducted in a controlled atmosphere, for example in a sterile environment with air circulation in laminar flow, followed by the labeling operation, single enveloping of each plate and packaging. This ensures that a plate is ready for use that has a shelf life of at least four months.

The plate production process is carried out in an automated production line in which the Agar Dilution phases are carried out in a clean room, then there is an in-line production phase in which a plurality of dispensers places the mixture in each well prepared in a standardized way followed by the controlled atmosphere packaging stage. In this way, hundreds of identical pieces are produced, which guarantees the end user both intra- and inter-laboratory repeatability.

The preferred production choice is to use plates similar to those of cellular microbiology with a standard number of wells (generally 12) which were easily mechanized and which were associated with a corresponding number of dispensers (as many dispensers as there were wells on each plate).

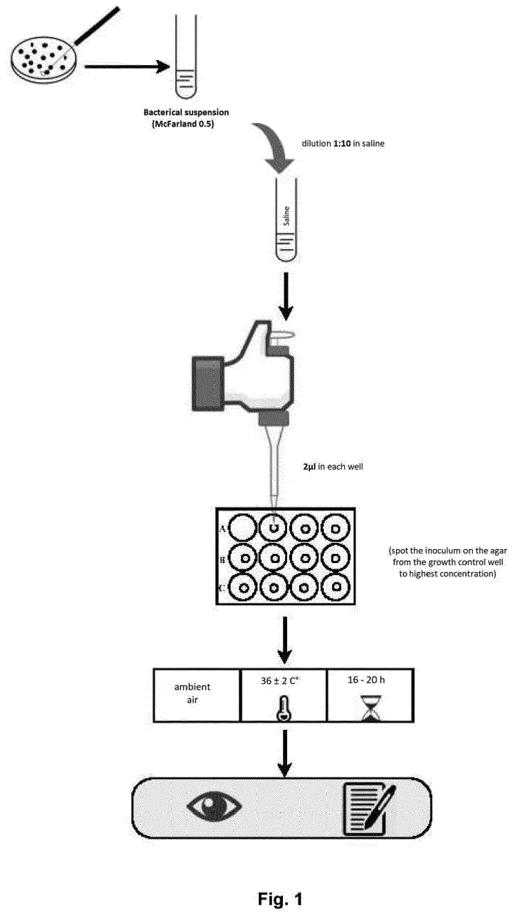

In accordance with the invention the MIC in agar is determined by the steps of inoculating a suspension of the bacterium to be tested in a plurality of agarized wells with serial two-fold dilutions of antibiotic. The test bacteria are inoculated on the agarized wells. These inoculated wells are incubated at 35-37°C overnight. The MIC is defined as the minimum antibiotic concentration that yielded complete inhibition of the bacteria on the wells. The steps are illustrated in Fig. 1 and Fig.s 2a-2e.

In the method of the present invention different bacterial strains such as *Staphylococcus spp., Enterococcus spp., Enterobactariaceae* and corresponding mixtures can be inoculated onto the surface of the agarized wells prepared according to Ref. (1) and containing various concentrations of antibiotic. The inoculated wells are incubated at about 35-37°C for a fixed time, preferably about 15-24 hours. Depending on the concentration of the antibiotic, bacterial growth results and is clearly visible on the wells. Generally, in the wells having the high concentration of antibiotic, the antibiotic inhibits the bacterial growth while on the wells having low antibiotic concentrations distinctly visible and typical growth is observed. The lowest antibiotic concentration which serves to inhibit the growth of the bacteria on the wells is defined as the minimal inhibitory concentration (MIC).

The present invention permits the assaying of the antibacterial activity of an antibiotic molecule with respect to a large varied number of bacteria in a very reliable way and high degree of reproducibility. It further permits the determination of the bactericidal activity of such antibiotic in a most simple and time-saving manner.

The molecules that can be testes can be selected in the group of antibiotics belonging to various classes, such as for example penicillins, cephalosporins, carbapenems, aminoglycosides, quinolones, fosfomycin, lincosamides, macrolides, glycopeptides, tetracyclines, azoles and echinocnidines.

The industrial production process gives stability to the thus prepared test kit, therefore a greater validity over time, i.e. shelf life can also be obtained. At the opposite, the production of in-house kits causes variability and instability of the substrates and consequently of the obtainable results of the tests performed by using such substrates.

The advantages of the kit of the invention are manifold and summarized below:
- Few manual operations borne by the end operator, relating only to the preparation of microorganisms to be tested;
- High speed of analysis, given that the end user of the device must not prepare the soil and antibiotic dilutions, but only open the package containing the plate ready for use;
- Reduced number of human errors, connected only with the above operations and not with the preparation of the agarized plates containing the antibiotic;
- Human errors further reduced because each well is individually identified and indexed on the plate;
- Reduced risk of contamination, connected only with the preparation of the microorganisms to be tested, since the end operator does not have to prepare every single agarized plate containing the antibiotic, but only has to open the package containing the plate ready for use;
- Reduced risk of contamination, further reduced by the need not to prepare each individual agarized capsule, because each analysis plate contains a plurality of wells already prepared for the various serial dilutions;
- Standardization of laboratory operations, thanks to the identity of the devices produced by the inventor.
- Use of a device that exactly reproduces the gold standard method recommended by the laboratory standards.
- Simplification of the analysis work and cost reduction due to all the above advantages.

All these advantages are obtained through the production process described above which is based on a simple and unique expedient, which is to carry out all production in a controlled and sterile environment. Not to mention the advantage that the mechanized production line allows to produce hundreds of identical pieces per batch, guaranteeing end users both intra- and inter-laboratory repeatability.

In the following an example to carry out the invention is given in order to more completely illustrate the invention but is in no wise to be construed as limitative of the scope thereof.

With reference to Fig. 1, it is schematically shown the aantibiotic Agar Dilution Workflow.

With particular reference to the Fig.s 2a-2e, the therein illustrated kit is a 12-well panel containing the antibiotic incorporated into an agar medium in different concentrations, i.e. 11 two-fold dilutions (0.25 - 256µg/ml). The procedure used to carry out the test is the agar dilution (AD)method for the antimicrobial susceptibility testing of the antibiotic and is according to the one recommended by CLSI and EUCAST (Ref. 1-4) standards but in a simpler and less time-consuming way and with highly reproducible results.

According to a preferred embodiment of the invention, the product package contains:
- 1 to 6 Systems (panels or plates) of antibiotic (panels individually packed in a plastic bag)
- Reading Template (a transparent sheet and a black one with printing of the antibiotic concentrations of each well in order to facilitate reading and interpretation).
- Instructions Sheet and Test Results Form (Reproduction in a table of the scheme of antibiotic concentrations contained in the agar dilution panel. The user of the kit notes the results on this form).

### ITEMS NECESSARY BUT NOT NECESSARILY INCLUDED IN THE KIT

- McFarland 0.5 Barium Sulphate Standard
- Physiological Solution

According to a preferred arrangement of the elements, the kit of the invention comprise at least one plate (or panel) with 12 wells containing an agar preparation, as previously indicated, organized in three rows A, B, C. Each row has four wells containing different antibiotic concentrations in the amounts indicated in table 1.

**Table 1**

| | **Antibiotic Concentration (µg/ml)** | | | |
|---|---|---|---|---|
| A | 256 | 128 | 64 | 32 |
| B | 16 | 8 | 4 | 2 |
| C | 1 | 0.5 | 0,25 | Control |

### Control: No antimicrobial agent in the well.

Each well preferably contains an amount of the agar composition ranging from 1 to 3 mL.

### PRINCIPLE OF THE METHOD

✔ All wells are inoculated with a standardized microbial suspension.
✔ After incubation in thermostat (as described in the following paragraph, for example at 36 ± 2°C for 16-20 hours in ambient air) the result is read and interpreted.

The antibiotic molecule is not for use directly with clinical or other specimens. The microorganism to be tested must first be isolated on a suitable non-selective culture medium. In case of mixed culture, selected colonies should be purified by sub-culturing according to known methods.

### TEST PROCEDURE

- Take a panel from its envelope and leave it at room temperature for 10 min.
- Prepare a suspension of the test organism using either the direct colony suspension or growth method.
- Standardize the suspension to the density of a McFarland 0.5 standard.
- Optimally within 15 min of preparation, dilute the adjusted suspension 1:10 in saline.
- Dispense 2µl of the inoculum solution over the agar surface (*) into each well (the final inoculums concentration should be approximately 10⁴ CFU per spot). Inoculate the growth-control well (no antimicrobial agent) first and then, starting with the lowest concentration, inoculate the other wells containing the different antimicrobial concentrations.
   (*) The agar composition is a Mueller Hinton Agar (MHA):
   Beef Extract 3.0 g; Acid Hydrolysate of Casein 17.5 g; Starch 1.5 g; G-6-P 0.025 g; Agar 17 g;
   Distilled Water 1000 ml;
   pH 7.3 ± 0.2
- Let the inoculated panel stand at room temperature until the moisture in the inoculum spots has been absorbed into the agar (i.e. until the spots are dry).
- Cover the panel with the lid provided and incubate at 36 ± 2°C for 16-20 hours in ambient air.

### READING THE RESULTS

At the end of the incubation period observe the growth in the wells and establish the MIC, i.e. the lowest concentration of antibiotic that inhibits visible growth. The results are read visually. Reading can be improved by placing the panel on a dark non reflecting surface.

The reading template provided with the kit allows an easy determination of the correct MIC result.

**Note:** Ensure that the panel is properly positioned before reading the results, the ABC on the template must match the letters (A), (B), (C) on the panel.

The growth-control well should be evaluated first. Make sure it is positive for growth. If not, check the viability of the colonies picked and repeat the test using a new panel and a microbial culture of recent growth.

Note the results on the Test Results Form included in the kit (copy as many form as necessary), which is a template to write down the test results.

### INTERPRETATION OF THE RESULTS

The MIC obtained should be interpreted according to current EUCAST or CLSI interpretative criteria.

### USER QUALITY CONTROL

Quality control of antibiotic is performed using for example the following reference strains:

| **Strain** | **MIC range** | |
|---|---|---|
| *Escherichia coli* | 0.5-2 | ATCC® 25922 |
| *Staphylococcus aureus* | 0.5-4 | ATCC® 29213 |
| *Pseudomonas aeruginosa* | 2-8 | ATCC® 27853 |
| *Enterococcus faecalis* | 32-128 | ATCC® 29212 |

### FACTORS THAT MAY INVALIDATE THE RESULTS

Contaminated culture; poor standardization of the inoculum; clinical material unsuitable; use of expired panels or expired supplementary reagents; non compliance with temperatures and times of incubation.

### PRECAUTIONS

The product does not contain hazardous substances in concentrations exceeding the limits set by current legislation and therefore is not classified as dangerous. It is nevertheless recommended to consult the safety data sheet for its correct use. The kit is a disposable device to be used for professional use only. The product must be used in the laboratory by properly trained personnel, using approved aseptic and safety methods for handling pathogenic agents.

### STORAGE

Store the kit at 2-8°C in the original packaging. Keep away from direct sunlight and direct heat. Do not use the panels beyond the expiry date indicated on the label. Eliminate without using if there are signs of deterioration.

### POSSIBLE KIT SET-UPS

| **Product** | **µg/ml** | **Packaging** |
|---|---|---|
| Agar Dilution panel | 0.25 - 256 | 6 test plates |
| Agar Dilution panel | 0.25 - 256 | 1 test plate |

### Agar Dilution Reading Guide

The Reading Guide is a guideline for the correct interpretation by the users of the device, which also contains examples of results.
- Record the MIC as the lowest concentration of antimicrobial agent that completely inhibit growth
- Disregard single colonies or faint haze caused by the inoculum

From the foregoing, it will be appreciated that specific embodiments of the technology according to the invention have been described herein for purposes of illustration, but that various modifications may be made without deviating from the present disclosure.

### REFERENCES

1. CLSI. Performance Standards for Antimicrobial Susceptibility Testing; 29th ed. CLSI Supplement M100S. Wayne, PA: Clinical and Laboratory Standards Institute; 2019.
2. CLSI. Methods for Dilution Antimicrobial Susceptibility Tests for Bacteria That Grow Aerobically; 11th ed. CLSI standard M07. Wayne, PA: Clinical and Laboratory Standards Institute; 2018.
3. The European Committee on Antimicrobial Susceptibility Testing. Breakpoint tables for interpretation of MICs and zone diameters. Version 9.0, 2019. http://www.eucast.org.
4. The European Committee on Antimicrobial Susceptibility Testing. Routine and extended internal quality control for MIC determination and disk diffusion as recommended by EUCAST. Version 9.0, 2019. http://www.eucast.org.

## Claims

1. A detection assay vessel in form of an agarized well having a U or V or flat bottom, depth 15-17mm, diameter 20-25mm, working volume 2-4ml, growth area 2-4cm², wherein the well contains an amount of a composition comprising agar and antibiotic prepared with doubled serial dilutions of antibiotic according to international guidelines (*Clinical and Laboratory Standards Institute,* January 2009, M07-A8, vol. 29 n.2) and sealed in a sterile environment to be ready for use.

2. A detection assay vessel according to claim 1 wherein the well contains an amount of the composition ranging from 1 to 3 ml.

3. A detection assay vessel according to claims 1-2 to perform an antibiotic susceptibility test in a sample with the agar dilution method.

4. A test kit for testing antibiotic susceptibility in a sample with the agar dilution method, wherein said test kit comprises one or more plates, each plate comprising at least one detection assay vessel in the form of an agarized well, wherein the well contains an amount of a composition comprising agar and antibiotic, each plate being prepared according to international guidelines (*Clinical and Laboratory Standards Institute,* January 2009, M07-A8, vol. 29 n.2) and sealed in a sterile environment to be ready for use.

5. A test kit according to claim 4 further comprising a Reading Template and instructions for use.

6. A test kit according to claim 4 further comprising a McFarland 0.5 Barium Sulphate Standard, an amount of Physiological Solution, a Reading Template and instructions for use.

7. A test kit according to any one of claims 4-6 comprising at least one 12-wells panel, each well containing an amount of antibiotic incorporated into an agar medium in different concentrations.

8. A test kit according to any one of claims 4-6 comprising at least one 12-wells panel, each well containing an agar preparation and the wells being organized in three rows A, B, C, each row having four wells containing different antibiotic concentrations in the amounts indicated in table 1 and a control with no antimicrobial agent in the well:
**Table 1**
| | **Antibiotics Concentration (µg/ml)** | | | |
|---|---|---|---|---|
| A | 256 | 128 | 64 | 32 |
| B | 16 | 8 | 4 | 2 |
| C | 1 | 0.5 | 0,25 | Control |

9. A test kit according to anyone of claims4-8 to determine the lowest antibiotic concentration which serves to inhibit the growth of bacteria on the wells.

10. A test kit according to any of claims 4-9 wherein the plates are 12-well cell microbiology plates.

11. A test kit according to any of claims 4-9 wherein antibiotics are selected in the following classes: penicillins, cephalosporins, carbapenems, aminoglycosides, quinolones, fosfomycin, lincosamides, macrolides, glycopeptides, tetracyclines, azoles, echinocnidines.

12. A process for preparing a plate comprising a plurality of agarized wells comprises the steps of:
- preparing the antibiotic solution and its dilutions;
- preparing the agarized soil (Mueller Hinton), sterilizing it and aliquoting it in 12 bottles;
- adding the respective antibiotic dilution to each bottle;
- distributing the antibiotic solutions in the respective wells of the device; said stages being conducted in a mechanized production line in a sterile environment and controlled atmosphere followed by the labeling operations, individual packing of each plate and packaging.

13. A process according to the previous claim in which the ready-to-use plate has a shelf life of at least four months.

14. A method for testing antibiotic susceptibility in a sample **characterized in that** it is carried out in the agarized wells according to claims 1-3 and in the test kit according to anyone of claims 4-11.

15. A method according to claim 14 comprising the following steps:
✔ (a) inoculating an amount of a liquid sample containing a bacterium at a given concentration in at least one detection assay vessel in form of an agarized well according to claims 1-3;
✔ (b) incubating the inoculated vessel of step (a) for 16-25 hours at 35-37°C; and
✔ (c) reading the incubated vessel of step (b) for a signal indication for a presence or an absence of bacteria.

16. A method according to anyone of claims 14-15 to determine the antibiotic susceptibility of a bacterial strain selected among *Staphylococcus spp., Enterococcus spp., Enterobactariaceae* and corresponding mixtures.

17. A method according to claim 15 comprising the following steps:
- preparing a plurality of agarized wells with serial two-fold dilutions of antibiotic concentration,
- inoculating test bacteria into the agarized wells,
- determining MIC activity on the basis of the presence or absence of the formation of macroscopic bacterial colonies after incubation of the inoculated wells for a fixed period of time and under predetermined laboratory conditions.
